# EUROPEAN PATENT APPLICATION

(11) **EP 2 915 497 A1**
(43) Date of publication of application: **09.09.2015**
(21) Application number: 15157542.0
(22) Date of filing: 04.03.2015
(51) Int. Cl.: A61B 17/80, A61B 17/74

(54) **Components for bone fixation devices**

(30) Priority: 06.03.2014 GB 201403946
(71) Applicant: National University of Ireland Galway, Galway (IE)
(72) Inventor: Harrison, Noel, Galway (IE); Bennett, Derek, Galway (IE); McDonnell, Pat, Galway (IE)
(74) Representative: Boyce, Conor

(57) **Abstract**

A dynamic hip screw comprises plate and a lag screw. The plate comprises a central ring portion from which a pair of oppositely directed wings extend, the plate having a concave medial surface and a convex lateral surface. The wings curve away from an axis transverse to a central axis of the ring portion. Each of the wings twist about the central axis as they extend away from the ring portion so that they extend in opposite directions away from the transverse axis. The lag screw comprises a shaft with a proximal end and a distal end for insertion into a bone. The distal end comprises a threaded section on which a main thread having a first thread depth is formed. A plurality of threads are defined on a leading surface of the main thread, each of the threads having a thread depth which is a fraction of the first thread depth.

## Description

### Field

The present application relates to components for bone fixation devices.

### Background Of The Invention

Hip fracture is the most common serious injury of older people and the number of fractures is predicted to double in the next 30 years. The annual number of hip fracture hospitalisations is 310,000 in the United States (AHRQ Publication No. 09-E013 August 2009) and 70,000 in the UK (National Hip Fracture Database National Report, 2011), with an associated treatment cost of $12.6 billion (US Pharm. 2011;36(10):) and £Stg1.4 billion, respectively.

Intertrochanteric hip fractures are generally treated with internal fixation devices which stabilise the fracture during the healing process. Referring to Figure 1, such devices include dynamic hip screws (DHS) such as disclosed in WO 2004/110292 and US2006/241606A1, Vachtenberg. These comprise a plate 10 including a number of screw holes 6-9 adapted to be attached to a femur or other bone (not shown); and a lag screw 2 adapted for insertion into a pre-reamed hole in a femoral head or other bone protrusion and rotationally lockable to the plate.

CN 202950735, Zeng, discloses an elastic dynamic hip joint screw where the lag screw includes a spring element allowing for controlled compression of the screw during healing of the fracture.

These devices are normally left in-situ during and after fracture healing. Surgical implantation of these devices involves at least one skin incision, at least as long as the DHS plate, and muscle dissection to gain access to the fractured bone. The plate is then slid and rotated into position through the incision. The incision size must therefore be large enough to allow instrumentation access and for the implant to be aligned with, and guided into the bone.

Reducing the invasiveness of these surgical procedures promotes rapid patient recovery, long term general wellbeing as well as management of health care costs.

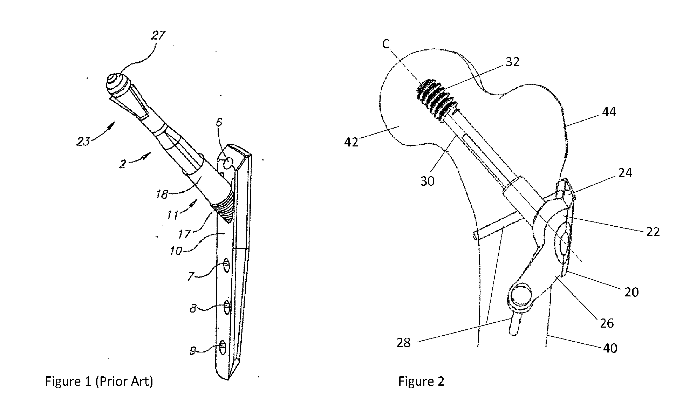

There are a number of hip fracture fixation devices on the market which are aimed at addressing the problem of minimal invasiveness and reduction of trauma to the patient. Examples include the Omega 3 Twin Hook system from Stryker, the PCCP system from Orthofix, the LCP-DHS system from Johnson&Johnson/Synthes, and Traumax from Integra Life Science. Each however, has a linear plate similar in construction that that shown in Figure 1.

At the same time, failure of fracture fixation devices continues to occur. For example, cut-out from the femoral head, whereby the physiological loading of the device causes the threaded end of the lag screw component to cut out through the femoral head and into the hip joint, is a significant problem. This necessitates a revision operation with a total hip replacement.

A number of devices on the market target the prevention of lag screw cut out. Among these is the X-Bolt, developed by X-Bolt Orthopaedics. The X-Bolt incorporates an expansion system that engages with the trabecular bone in the femoral head and is deployed once the bolt is in place. However difficulties can arise in retracting this implant if the bone mass has moved or remodelled in the time following implantation.

It is an object of the present invention to mitigate these problems with the prior art.

### Summary

Accordingly, a first aspect of the present invention provides a plate for a bone fixation device as claimed in claim 1.

Fixation devices including for example, dynamic hip screws including the plate can be implanted through only one relatively small incision, typically 3 cm length. As outlined above, a smaller single incision results in reduced trauma and blood loss for the patient.

The plate requires as few as two fixing screws. Along with the smaller single incision, using a minimal number of fixing screws provides a simple installation method with standard instrumentation. This in turn results in a shorter operating time compared to a standard DHS-plate.

The plate can provide an optimised angle for fixing screws, resulting in a stronger fixation compared to existing DHS-plate devices - this can be particularly beneficial for patients with very osteoporotic bone.

In the event that the device must be removed, a smaller incision and less invasive operation procedure is required compared to a standard DHS-plate device.

A second aspect provides a lag screw for a bone fixation device, the lag screw comprising a shaft with a proximal end and a distal end for insertion into a bone, the distal end comprising a threaded section on which a main thread having a first thread depth is formed, wherein a plurality of threads are defined on a leading surface of said main thread, each of said threads having a thread depth which is a fraction of said first thread depth.

Preferably, the lag screw includes approximately 6 threads defined on said leading surface.

Preferably, said threads are formed by one of machining or rapid prototyping including DMLS.

Preferably, each of said main thread and said plurality of threads comprise respective helical threads, each having the same pitch.

Forming micro-scale threads on the lag screw increases the surface area of the lag screw for bone attachment and load distribution as well as increasing the friction of the lag screw under push out loads. Thus, the likelihood of lag screw cut out is reduced.

The lag screw can be implanted and removed in the same way as current lag screws i.e. by a screwing action. This means that re-training for surgeons to employ, for example, a dynamic hip screw including such a lag screw is minimal, so facilitating its clinical adoption.

Preferably, the lag screw further includes a resilient compression device extending from said proximal end of said lag screw. Further preferably, said compression device comprises a coil spring. The compression device can be arranged to locate within a barrel.

In embodiments where a resilient compression component is provided within the lag screw, compression of a fracture site can be controlled to provide optimum local bone healing conditions. This can increase the rate of fracture healing and reduce the risk of limb shortening.

In a third aspect of the invention there is provided a dynamic hip screw including a plate according claim 1, a lag screw according to the second aspect and a barrel for connecting said plate to said lag screw.

The lag screw can be arranged to slidably locate within the barrel. Further preferably, the lag screw is arranged to be rotatably fixed relative to said barrel.

Decoupling of the barrel from the plate allows freer manoeuvrability during the insertion of the device, thereby significantly reducing the size of the required incision.

### Brief Description Of The Drawings

The present application will now be described, by way of example, with reference to the accompanying drawings in which:
Figure 1 shows a conventional dynamic hip screw (DHS);
Figure 2 shows a dynamic hip screw (DHS) according to a first embodiment of the present invention in-situ on a left femur;
Figure 3 is an assembled perspective view of the DHS of Figure 2;
Figure 4 is an exploded perspective view of the DHS of Figure 2;
Figures 5-7 are further rotated views of the DHS of Figure 2;
Figure 8 illustrates in detail an exemplary fixing mechanism connecting the plate to a lag screw barrel for the DHS of Figure 2;
Figure 9 shows in detail a multi-scale thread formed at the end of the lag screw component of the DHS of Figure 2;
Figure 10 is a cross-sectional view of a thread of the lag screw of Figure 9; and
Figures 11 and 12 are exploded and cross-sectional views respectively of a lag screw and barrel arrangement according to a second embodiment of the present invention.

### Detailed Description Of The Drawings

Referring now to Figure 2, a DHS comprises a plate 20 and a lag screw 30 according to a first embodiment of the present invention. The lag screw 30 comprises a threaded head 32 and is inserted head-first into the femoral head 42 in a conventional fashion, with its threaded head 32 anchoring the lag screw in place as will be described in more detail later.

The plate 20 comprises a central ring portion 22 having a central axis from which a pair of wings 24 and 26 extend. When fitted to the femur, the centre of the ring portion 22 of the plate 20 lies co-axial with the axis (C) of the lag screw.

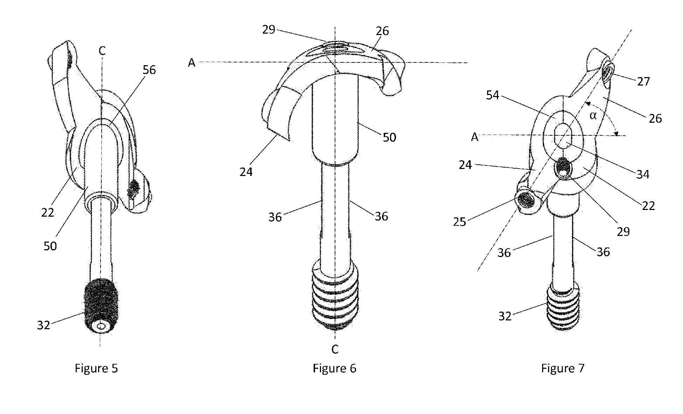

The medial surface of the plate 20 has a concave curvature with each of the wings curving away from an axis (A) transverse to the central axis (C) of the ring portion towards the sagittal plane - this curvature can be seen more clearly in Figure 6. The radius of curvature is between approximately 15-20mm but this does not have to be circular or uniform across the extent of the plate, nor do the centres of curvature of the wings 24, 26 have to be coincident. In the illustrated embodiment, for example, the radius of curvature of the wing 24 is approximately 20mm while that of the wing 26 is tighter at approximately 15mm.

Thus, as shown in Figure 2, when fitted to a lateral surface of the femur 40 typically below the trochanter 44, the plate conforms to the surface of the femur extending posterior and anterior from the ring portion. (It should therefore be appreciated that in order to be fitted across a range of bone sizes, the curvature and extent of the wings may vary across a range of plates.)

As well as curving, each of the wings 24, 26 also twist about the central axis of the plate so that when fitted against the bone surface, the posterior extending wing 24 extends upwardly while the anterior extending wing 26 extends downwardly. (In alternative embodiments, this twisting could be reversed so that the posterior extending wing 24 extends downwardly while the anterior extending wing 26 extends upwardly.) In the illustrated embodiment, Figure 7, the wings twist with a helical angle α of approximately 45°, although angles from 30° to 60° could be employed according to the particular application. Nonetheless, it will be appreciated that the twisting angle of each wing 24, 26 can differ and also that the twisting angle can vary along the extent of each wing in order to better conform to the bone surface.

In the illustrated embodiment, a threaded hole 25, 27 is provided at the distal end of each wing 24, 26 respectively. In the illustrated embodiment, the centre to centre distance between the holes is approximately 62mm with the wings having a maximum span of approximately 68mm. In the embodiment, the plate 20 is secured to the femur 40 using standard locking screws 28, Figure 2, which are threaded at the head of the screw (not shown) as well as along the shaft of the screw. The threaded head of the screws engage with the corresponding thread in the plate holes 25, 27, making plate fixation more resistant to screw loosening and pull-out. Nonetheless, it will be appreciated that in variations of the illustrated embodiment, nonthreaded holes 25, 27 can be employed with standard fixing screws.

In variants of the illustrated embodiment, each wing 24, 26 could incorporate more than one hole to allow either more than one screw to fix the plate 20 to the femur 40 or for a given screw to be inserted in an optimal fixing location.

Where multiple holes are provided in each wing 24, 26, the twisting of the wings means that the fixing screws can be oriented at a wider range of angles compared to conventional DHS-plate devices to ensure stronger plate fixation to the bone and/or potentially a smaller number of fixing screws can be used.

Again, the twisted and curved profile of the plate facilitates minimal invasiveness since access for insertion of the locking screws 28 can be achieved via the incision for the plate by manipulating the window of access during surgery. This would not be possible for a standard DHS plate since the fixing screws are arranged linearly whereas for the plate 20, the curvature of the plate means that the fixing screw locations are more easily accessible from a small incision.

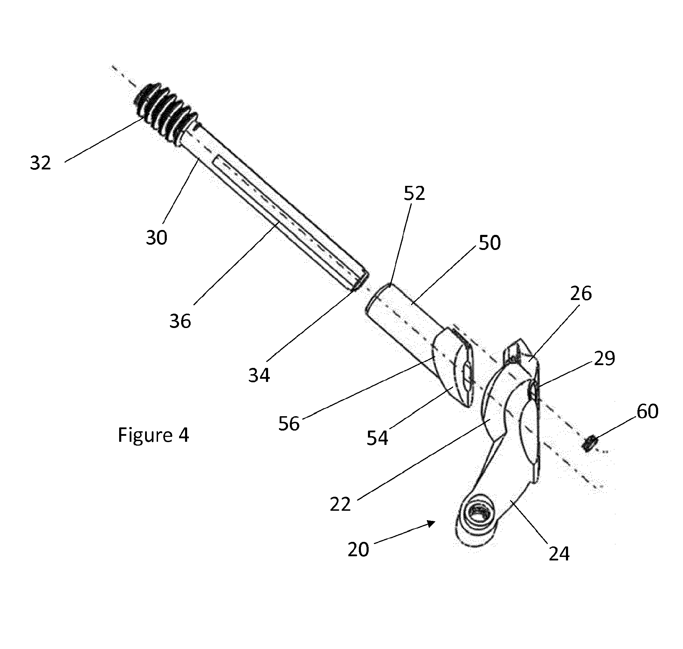

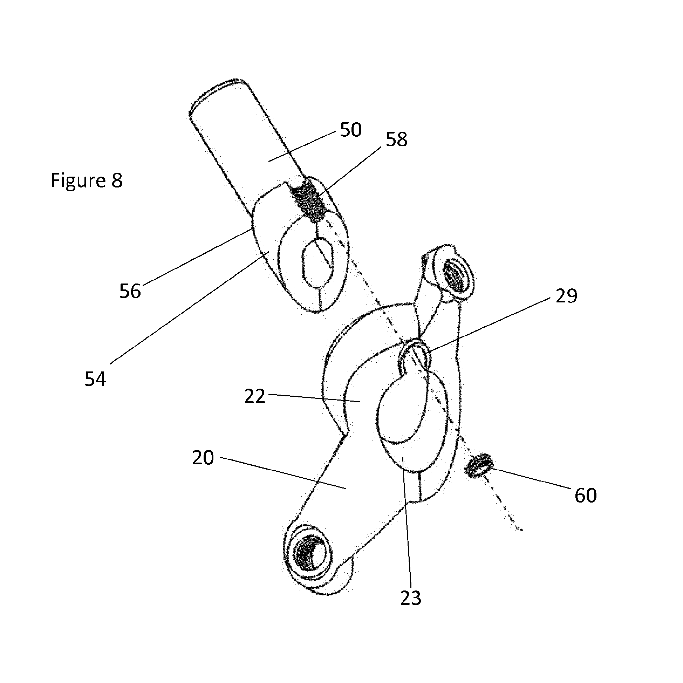

In the embodiment of Figures 2-8, the plate 20 is slidably connected to the lag screw 30 via a separate barrel 50. The barrel 50 comprises a generally hollow cylinder having an internal cross-section complementary to and shaped to accommodate a proximal end 34 of the lag screw 30 through its open distal end 52, allowing the barrel to slide relative to the lag screw 30. In the illustrated embodiment, the outer surface of the proximal end 54 of the barrel tapers down from a raised shoulder 56 towards the proximal end. The internal surface 23, Figure 8, of the ring 22 is formed with a corresponding taper. In embodiments employing such a tapered connection, a clinician first implants the lag screw 30, followed by the barrel 50, and finally the plate 20 is fitted to the barrel 50. In an alternative embodiment, the tapers could be reversed and in this case, the barrel would pass through an *in situ* plate locating over the proximal end 34 of the lag screw 30 until the tapered surfaces of the barrel and plate mate to fix these parts together.

In any case, this separate plate-barrel arrangement allows for a more minimally invasive operation since the plate can be slid through a small incision, independently of the barrel. This is not the case with DHS-plate devices where the plate and barrel form a single component.

Referring back to the drawings, the lateral surface of the plate 20 is convex with similar curvature to the medial surface with the plate having a generally uniform thickness of between about 3mm and 15mm and preferably approximately 6mm. The proximal surface 54 of the barrel is similarly curved, so that when the barrel and plate mate, a generally smooth lateral surface is provided.

In order to maintain a rotational alignment of the plate 20 and lag screw 30, a pair of flats 36 are formed or machined on opposite faces of the lag screw 30 and extend from the proximal end 34 along the length of the screw towards, but stopping short of the distal threaded head 32. The internal surface of the barrel has a complementary cross-section with corresponding flat surfaces, so that when the lag screw 30 locates in the barrel, it can slide to a limited extent within the barrel, but is prevented from rotating within the barrel.

The flats 36 also provide an engagement surface for a screw tool (not shown) to fix the lag screw 30 in position as well as providing a fiduciary reference for determining the angular position of the inserted lag screw. As will be appreciated from the description below, the inserted angular position of the lag screw can in turn determine the angle of fixation of the plate 20.

The embodiment of Figures 2-8 includes an additional mechanism for fixing the barrel to the plate. Referring to Figure 8, an off-axis blind hole 29 is formed in the plate ring 22, the hole 29 being open at the lateral surface of the plate 20. The hole 29 is also open along one side into the open centre of the plate ring 22. Partial threads 58 are machined on the external surface of the tapered surface 54 of the barrel with the same radius of curvature as the hole 29. The plate is fitted to the barrel with the threads 58 and hole 29 aligning rotationally. A small grub screw 60 can then be inserted into the hole 29 and threaded until it bottoms against the bottom of the blind hole 29. At this stage rotating the screw 60 can clamp the barrel 50 to the plate.

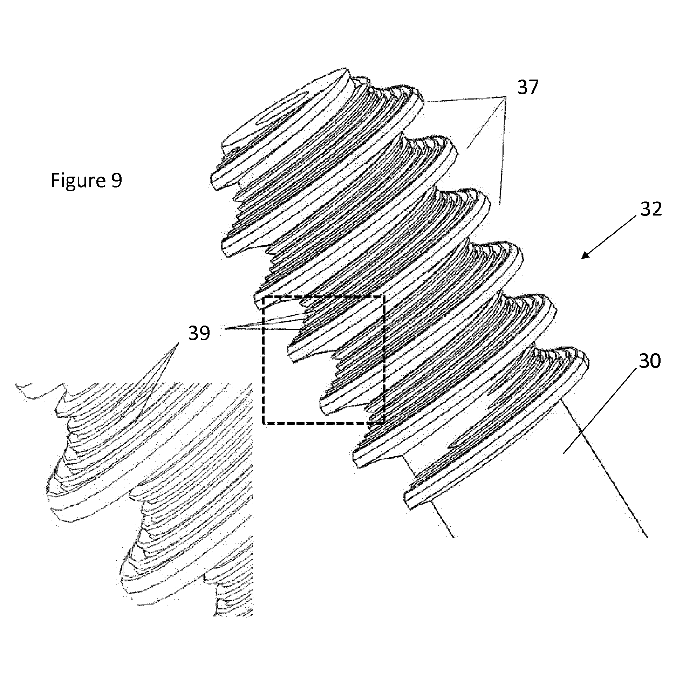

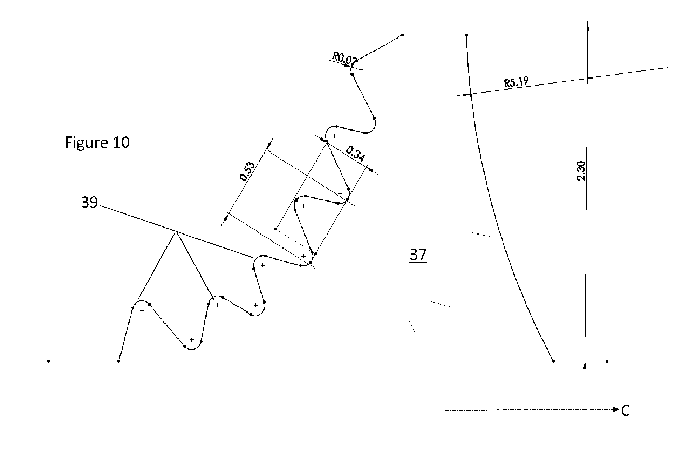

Turning now to Figures 9 and 10, which illustrate the threaded head 32 of the lag screw in detail. As in conventional lag screws, a raised main helical thread 37 is formed on the shaft of the lag screw. This is typically formed by machining down a larger diameter shaft in a conventional fashion. In the embodiment of Figures 9 and 10, a series of N micro threads 39, in the illustrated example, N=6, are also formed on the leading face of the main screw thread 37. Each micro thread 39 has a thread depth which is a fraction of the main thread depth and each is displaced a fraction, f(1/N), of the main thread depth from an adjacent micro thread. These micro threads can be formed, for example, by machining away the leading face of the main screw thread 37 as in the illustrated example where the multiple thread starts are angularly displaced from one another; or by forming micro scale ridges along the machined leading surface of the main screw thread, using for example a rapid prototyping process such as direct metal laser sintering (DMLS) or electron beam melting described in US 2010/298950.

Figure 10 shows a typical set of dimensions for a machined set of micro threads, the dimensions being given in millimeters.

The micro threads 39 increase the surface area of the screw thread 37, offering a larger surface for bone attachment to occur on and a greater surface area over which to transfer femoral head loading. This reduces the local pressure on, for example, the trabecular bone inside the femoral head. The threads 39 also act to increase the friction of the lag screw against the trabecular bone, when the hip is loaded.

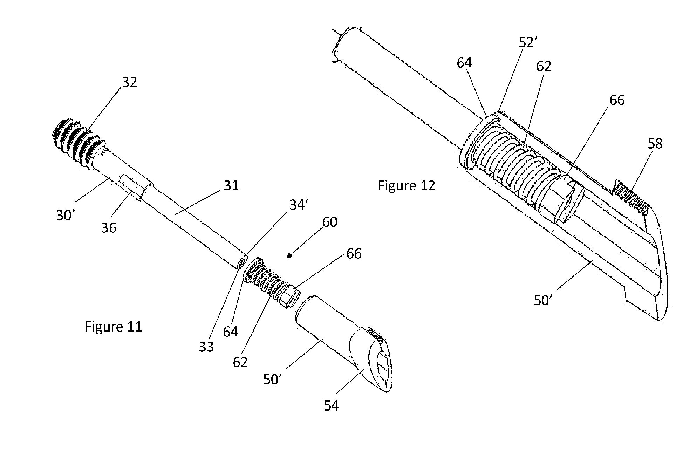

Referring now to Figures 11 and 12, in the first embodiment, the lag screw 30 can slide in the barrel 50, allowing dynamic compression at the fracture site to aid healing. In a second embodiment of the lag screw, a compression optimisation device 60 is fitted to the proximal end of the lag screw 30' to regulate the compression at the fracture site. This device contains a damping mechanism, in this case a spring 62 that extends to cause the lag screw 30' to absorb and transmit some of the hip load directly to the plate 20, if the compression of the fracture site exceeds a predefined threshold. The spring is fitted between a collar 64 and a cap 66. The collar 64 sits in a recess formed in the mouth of the distal end 52' of the barrel with the spring 62 extending into the barrel. The cap 66 has the same cross-sectional profile as the internal surface of the barrel 50'. This again is non-circular with flat side walls so tending to limit rotational movement of the mechanism 60 within the barrel. The proximal portion 31 of the lag screw is machined down to a circular section narrow enough to slide through the collar 64 into the spring 62 and to bear against the cap 66. Thus, in use, the spring 62 extends in proportion to the compression load between the lag screw and plate.

In order to rotationally fix the cap 66 and lag screw 30' relative to one another, a bolt or screw (not shown) can be fitted through the cap from its proximal surface and screwed into a socket 33 formed in the proximal surface of the lag screw 30'. As such, the lag screw 30' and damping mechanism 60 can be first assembled before being implanted, with the barrel 50' and plate 20 fitted to the implanted assembly as in the previously described embodiment.

The stiffness profile of the compression optimisation device 60 can be calibrated to give optimum compression of the fracture surfaces for bone healing to occur. The profile can be set between about 10-5000N/mm and preferably around 600N/mm.

In variants of the illustrated embodiment, the spring 62 may also comprise two or more spring elements of different stiffness.

In still further variants of the illustrated embodiment, the spring could be integrally formed in the lag screw, by for example wire cutting alternating transverse slots along the proximal end of the lag screw.

As will be appreciated, with the lag screw of the second embodiment, compression at the fracture surface is maintained at an optimum magnitude for bone healing to occur, and excessive compaction of the fracture surfaces leading to limb shortening is minimised.

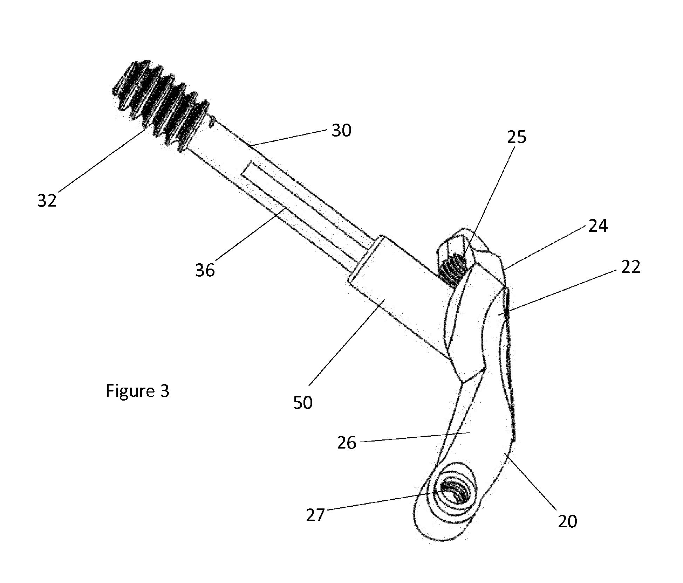

In variants of the embodiment of Figures 3-8 and 11-12, the plate and barrel can be connected using a threaded joint or indeed any suitable rotational connection, for example, a bayonet type fit. If a threaded joint is used between the barrel 50, 50' and the plate 20, the plate is placed over the femur before the barrel is threaded through the plate. Both assembly methods result in a rigid construct.

For each of the above described embodiments, production costs are comparable to existing devices, since standard production methods and materials will be used.

It will be seen that the construct and orientation of the plate 20 means that the locking screws 28 can be optimally oriented at different angles and from positions on the femur which are anterior and posterior to the lag screw 30 entry point. This allows better fixation to the patient's bone and a higher overall strength of the construct. This can also result in fewer locking screws being required to achieve stable fixation compared to standard DHS-plate devices.

The words comprises/comprising when used in this specification are to specify the presence of stated features, integers, steps or components but does not preclude the presence or addition of one or more other features, integers , steps, components or groups thereof.

## Claims

1. A plate (20) for a bone fixation device, the plate comprising a central ring portion (22) from which a pair of oppositely directed wings (24,26) extend, the plate having a concave medial surface and a convex lateral surface, with said wings curving away from an axis transverse (A) to a central axis (C) of said ring portion, wherein each of said wings twist (α) about said central axis as they extend away from said ring portion so that they extend in opposite directions away from said transverse axis.

2. A plate according to claim 1 wherein said radius of curvature of said wings is between approximately 15mm and approximately 20mm.

3. A plate according to claim 1 wherein the centre of curvature of said wings is non-coincident.

4. A plate according to claim 1 wherein the curvature of said wings is non-circular.

5. A plate according to claim 1 wherein the curvature of said wings is non-uniform.

6. A plate according to claim 1 wherein a twisting angle (α) of said wings is between approximately 30° and approximately 60° and preferably, the angle is approximately 45°.

7. A plate according to claim 1 wherein the twisting of said wings is approximately helical.

8. A plate according to claim 1 wherein the twisting angle of said wings is non-uniform.

9. A plate according to claim 1 wherein at least one fixing hole (25, 27) is provided in each wing of said plate.

10. A plate according to claim 1 wherein said ring portion comprises an open inner surface (23) arranged to accommodate and connect said plate to a complementary barrel (50).

11. A plate according to claim 10 wherein said inner surface is either threaded, tapered or otherwise arranged to rotationally connect said plate to a complementary barrel.

12. A plate according to claim 10 wherein said ring portion includes a partial blind hole (29) which opens into said inner surface, said partial blind hole being arranged to align with a complementary partially threaded portion (58) formed on said barrel.

13. A plate according to claim 1 wherein the wings extend a distance of approximately 68mm.

14. A plate according to claim 1 where the plate is approximately 6mm in thickness.

15. A dynamic hip screw including a plate according claim 1, a lag screw (30) and a barrel (50) for connecting said plate to said lag screw.
